# EUROPÄISCHE PATENTSCHRIFT

(11) **EP 2 133 037 B1**
(45) Veröffentlichungstag und Bekanntmachung des Hinweises auf die Patenterteilung: **15.09.2010**
(21) Anmeldenummer: 08157837.9
(22) Anmeldetag: 09.06.2008
(51) Int. Cl.: A61B 19/00, A61B 6/08

(54) **Patientenmarker**
Patient marker
Marqueur de patients

(43) Veröffentlichungstag der Anmeldung: 16.12.2009
(73) Patentinhaber: Renate Beck GmbH, 94113 Tiefenbach (DE)
(72) Erfinder: Beck, Heinz, 94113, Tiefenbach (DE)
(74) Vertreter: Gustorf, Gerhard

(56) Entgegenhaltungen:
- US-A- 5 193 106
- US-A- 5 260 985
- US-A- 5 702 128
- US-A- 5 743 899
- US-A1- 2007 280 406

## Beschreibung

Die Erfindung betrifft einen sogenannten Patientenmarker, d. h. ein Mittel zum Markieren von Körperteilen für radiologische Behandlungen oder Untersuchungen, beispielsweise CT-Aufnahmen.

Für die genannten Zwecke war es bisher üblich, die von Laserstrahlen vorgegebenen Markierungspunkte der betreffenden Körperteile mittels eines Farbstiftes zu kennzeichnen. Um diese Markierungen, im allgemeinen Kreuze, im Röntgenbild sichtbar zu machen, wurden auf diese Bleidrähte geklebt, beispielsweise mittels eines Heftpflasters. Die Bleidrähte ergeben aufgrund der Röntgendichte des Materials im Röntgenbild eine helle Markierung. Schwermetalle wie Blei sind allerdings giftig. Derartige Verfahren, die unmittelbar am Patienten durchgeführt werden, sind sehr zeitaufwendig.

Wesentliche Nachteile dieser bekannten Verfahren bestehen darin, dass sich die Farbe des Markierungsstiftes, im allgemeinen eines Filzstiftes, durch Schweiß oder Feuchtigkeit auf Kleidungsstücke und Bettwäsche überträgt und nach wenigen Tagen auf der Haut verläuft und verblasst, so dass die Markierungen dann nachgezogen werden müssen. Bei diesem Nachziehen sind Positionsfehler nicht zu vermeiden, so dass die Markierung dann ungenau wird. Im übrigen dringt die Farbe des Markierungsstiftes in die Haut ein und kann Reizungen verursachen. Nicht zuletzt fallen die von Hand aufgebrachten Markicrungskreuze in Form und Winkel unterschiedlich aus, was eine Quelle für weitere Ungenauigkeiten darstellt.

Gemäß einem anderen bekannten Verfahren werden Pflaster verwendet, die eine kleine Metallkugel tragen, um den Markierungspunkt zu kennzeichnen. Ein Nachteil besteht hierbei darin, dass bei Schichtaufnahmen im CT der Markierungspunkt maximal auf nur einem Bild erscheinen kann. Im ungünstigsten Fall liegt die Markierung zwischen zwei Aufnahmen und ist dann überhaupt nicht sichtbar.

Gegenstand der US-A 5 743 899 ist ein Patientenmarker mit den Merkmalen des Oberbegriffs des Patentanspruchs 1. Hierbei ist eine bandförmige Trägerfolie für mehrere, unterschiedlich geformte Markierungselemente vorgesehen, die je nach Bedarf einzeln auf ein Körperteil aufgebracht werden. Zu diesem Zweck muss zuvor ein Randstreifen der Trägerfolie abgezogen werden, um die Markierungselemente an einem Teilbereich freizulegen.

Der Erfindung liegt die Aufgabe zugrunde, einen Patientenmarker der im Oberbegriff des Anspruchs 1 umrissenen Gattung zur Verfügung zu stellen, der einfach, präzise und reproduzierbar auf dem betreffenden Körperteil angebracht werden kann und über längere Zeit erhalten bleibt, ohne dass die Gefahr besteht, dass die Markierung durch Feuchtigkeit oder Körperschweiß verblasst oder in die Kleidung eindringen kann.

Zur Lösung dieser Aufgabe ist vorgesehen, dass die Folie auf ihrer mit Klebstoff beschichteten Seite von einer abziehbaren Silikon-Schutzfolie abgedeckt ist, während sie mit ihrer gegenüberliegenden Seite fest mit einem flachen Träger verbunden ist, der im Bereich der Folie eine Aussparung aufweist.

Das gemäß der Erfindung ausgebildete Mittel zum Markieren von Körperteilen lässt sich sehr genau und relativ dauerhaft auf dem betreffenden Körperteil anbringen, ohne dass bisher übliche Filzstifte oder dergleichen notwendig sind. Der Patientenmarker kann sehr präzise auf den von Laserstrahlen definierten Markierungspunkten positioniert werden, weil das Markierungs-element, im allgemeinen ein Kreuz, durch die Folie sichtbar ist.

Es ist von Vorteil, wenn die Folie innerhalb der Aussparung des Trägers, der aus einem Kunststoff-, Papier- oder Kartonabschnitt bestehen kann, eine ringsum umlaufende Perforierung aufweist.
Nach der Positionierung kann dann der Anwender durch Daumendruck die Folie mit dem Markierungselement vom Träger im Durchdrückverfahren abtrennen und glatt auf dem Körperteil fixieren.

Es ist vorteilhaft, wenn nach einem weiteren Merkmal der Erfindung das Markierungselement zwischen der durchsichtigen Klebstoffschicht und der Folie eingeschlossen ist.

Mit der Erfindung ergibt sich die Möglichkeit, derartige Patientenmarker in Serie herzustellen und Kliniken und Anwendern einsatzbereit zur Verfügung zu stellen.

Nach einem weiteren Merkmal der Erfindung besteht das Markierungselement aus einer Farbpaste, die Bariumsulfat enthält, wodurch die Markierung in Röntgenaufnahmen sehr gut sichtbar gemacht wird. Alternativ besteht die Möglichkeit, das Markierungselement aus Metallfäden, z. B. Silberfäden herzustellen. Gemäß einer anderen Alternative werden für das Markierungselement Metallkettchen verwenden, die aufgrund ihrer Gelenkstellen eine ausgezeichnete Anpassung an Körperrundungen gewährleisten.

Weitere Merkmale und Vorteile der Erfindung ergeben sich aus den Unteransprüchen und aus der folgenden Beschreibung eines Ausführungsbeispiels, das in der Zeichnung dargestellt ist. Es zeigen:
Figur 1 die Vorderansicht eines Patientenmarkers gemäß der Erfindung,
Figur 2 in teilweise Explosionsdarstellung einen Querschnitt durch den Patientenmarker der Figur 1,
Figur 3 die dem Körperteil zugewandte Rückseite des Patientenmarkers und
Figur 4 die Teildarstellung eines Armes mit aufgebrachtem Patientenmarker.

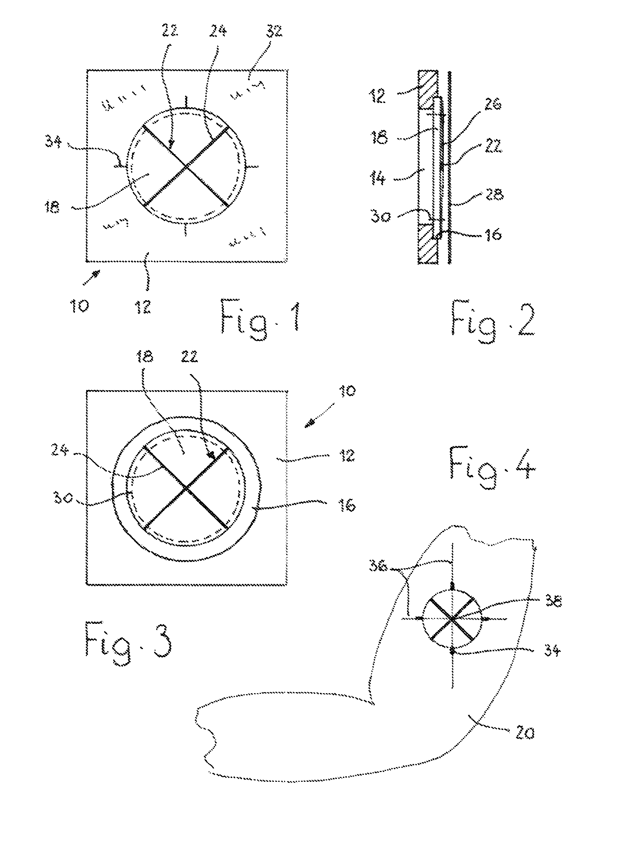

Der in den Figuren 1 bis 3 gezeigte Patientenmarker 10 besteht aus einem hier quadratischen Träger 12 aus Kunststoff, Papier oder einem dünnen Kartonabschnitt, der in der Mitte eine kreisförmige Aussparung 14 hat. In einer die kreisförmige Aussparung 14 umgebenden, flachen, ringförmigen Einprägung 16 ist eine biegsame, durchsichtige Folie 18 befestigt, die aus einem atmungsaktiven und hautfreundlichen Kunststoff besteht, beispielsweise eine PE-Folie.

Auf die zum Körperteil 20 weisende Seite der Folie 18 ist ein Markierungselement 22 aufgebracht, das vorzugsweise aus einem Kreuz mit zwei Schenkeln 24 besteht, die sich unter einem rechten Winkel im Mittelpunkt der kreisförmigen Aussparung 14 schneiden. Das Markierungselement 22 besteht im dargestellten Ausführungsbeispiel aus einer Farbpaste, die Bariumsulfat enthält und mittels Siebdruck, Tampondruck oder Sprühtechnik auf die Folie 18 aufgebracht ist. In der Schnittdarstellung der Figur 2 ist das Markierungselement 22 aufgrund der durch den Kreuzungspunkt verlaufenden Schnittebene nur als eine kurze Gerade zu erkennen.

Alternativ kann das Markierungselement 22 aus Metall bestehen, beispielsweise aus zwei die Schenkel 24 bildenden Silberfäden. Eine weitere Möglichkeit besteht darin, anstelle der Metallfäden dünne Metallkettchen zu verwenden.

In Figur 2 ist angedeutet, dass die Folie 18 auf ihrer zum Körperteil 20 weisenden Seite mit einer Klebstoffschicht 26 versehen ist, die das Markierungselement 22 abdeckt, so dass dieses zwischen der Klebstoffschicht 26 und der Folie 18 eingeschlossen ist. Die durchsichtige Klebstoffschicht 26 ist ebenso wie die Folie 18 hautfreundlich und so ausgebildet, dass sie mehrere Tage auf der Haut des Körperteils 20 haftet. Als Material für den Kleber eignet sich insbesondere ein Kleber auf Acrylatbasis.

Auf ihrer mit Klebstoff 26 beschichteten Seite ist die Folie 18 von einer abziehbaren Silikon-Schutzfolie 28 abgedeckt, die in Figur 2 im bereits abgezogenen Zustand .vor dem Anbringen des Patientenmarkers 10 auf dem Körperteil 20 dargestellt ist.

Gemäß der Erfindung ist es von besonderem Vorteil, dass die Folie 18 innerhalb der Aussparung 14 eine ringsum laufende Perforierung 30 hat. Damit ist eine Solltrennstelle geschaffen, so dass der Anwender nach dem Positionieren des Patientenmarkers 10 die Folie 18 mit dem Markierungselement 22 durch Daumendruck abtrennen und an dem Körperteil 20 fixieren kann.

Figur 1 zeigt schließlich, dass der Träger 12 auf seiner Außenseite, die beim Anbringen vom Körperteil 20 wegweist und mit einer Beschriftung 32 versehen sein kann, vier Referenzlinien 34 trägt, die auf den Winkelhalbierenden des kreuzförmigen Markierungselementes 22 liegen und somit zu dem jeweiligen Schenkel 24 des Kreuzes einen Winkelabstand von 45° haben.

Bei CT-Aufnahmen wird nach Abziehen der Schutzfolie 28 der Patientenmarker 10 auf das in Figur 4 angedeutete Körperteil 20 gelegt, wobei die vier Referenzlinien 34 genau auf den beiden Laserlinien 36 liegen, die sich unter einem rechten Winkel schneiden und den Markierungspunkt in ihrem Schnittpunkt 38 kennzeichnen. Sodann wird die Folie 18 mit Daumendruck durchgedrückt, wobei sie sich entlang der Perforierung 30 vom Träger 12 löst und am Körperteil 20 glattgedrückt wird.

Da gemäß Figur 4 die beiden Schenkel 24 des kreuzförmigen Markierungselementes 22 gegenüber den Laserlinien 36 um 45° gedreht sind, entstehen bei Schichtaufnahmen jeweils zwei sichtbare Punkte, deren Abstand sich bis zum Schnittpunkt 38 verringert, so dass dort dann nur noch ein Punkt auf der Aufnahme entsteht. Anschließend vergrößert sich dieser Abstand wieder, so dass der Anwender genau die Lage der jeweiligen Schichtaufnahme rekonstruieren kann.

Bei der Strahlentherapie wird ebenso verfahren wie bei CT-Aufnahmen. Der einzige Unterschied besteht darin, dass das aufgedruckte Markierungselement 22 am Laserstrahl 36 ausgerichtet und fixiert wird.

Bei älteren Bestrahlungsgeräten reicht die Röntgendichte der Patientenmarker auf Bariumsulfatbasis für Kontrollaufnahmen nicht aus. Hier wird die Markierung 22 mit Bariumsulfat durch Metallkettchen oder Metallfäden ersetzt. Diese Patientenmarker ergeben auch bei Kontrollaufnahmen in älteren Bestrahlungsgeräten gut sichtbare Markierungen auf dem Röntgenbild.

## Patentansprüche

1. Mittel zum Markieren von Körperteilen für radiologische Behandlungen oder Untersuchungen mit einem an dem Körperteil anbringbaren Markierungselement (22), das eine röntgendichte Substanz enthält und auf einer durchsichtigen, biegsamen Folie (18) angebracht ist, die auf einer Seite mit einem am Körperteil (20) haftenden Klebstoff (26) beschichtet ist, **dadurch gekennzeichnet, dass** die Folie (18) auf ihrer mit Klebstoff (26) beschichteten Seite von einer abziehbaren Silikon-Schutzfolie (28) abgedeckt ist, während sie mit ihrer gegenüberliegenden Seite fest mit einem flachen Träger (12) verbunden ist, der im Bereich der Folie (18) eine Aussparung (14) aufweist.

2. Mittel nach Anspruch 1, **dadurch gekennzeichnet, dass** die Folie (18) innerhalb der Aussparung (14) eine ringsum umlaufende Perforierung (30) aufweist.

3. Mittel nach Anspruch 1 oder 2, **dadurch gekennzeichnet, dass** die Aussparung (14) eine Kreisform hat.

4. Mittel nach einem der vorhergehenden Ansprüche, **dadurch gekennzeichnet, dass** der Träger (12) aus einem Kunststoff-, Papier- oder Kartonabschnitt besteht.

5. Mittel nach einem der vorhergehenden Ansprüche, **dadurch gekennzeichnet, dass** das Markierungselement (22) zwischen der durchsichtigen Klebstoffschicht (26) und der Folie (18) eingeschlossen ist.

6. Mittel nach einem der vorhergehenden Ansprüche, **dadurch gekennzeichnet, dass** der Träger (12) rechteckig, vorzugsweise quadratisch ist.

7. Mittel nach einem der vorhergehenden Ansprüche, **dadurch gekennzeichnet, dass** das Markierungselement (22) aus einem Kreuz besteht, dessen beide Schenkel (24) rechtwinklig aufeinander stehen und sich im Mittelpunkt der Aussparung (14) schneiden.

8. Mittel nach Anspruch 7, **dadurch gekennzeichnet, dass** am Außenrand der Aussparung (14) der Träger (12) vier Referenzlinien (34) aufweist, die auf den Winkelhalbierenden des Kreuzes liegen.

9. Mittel nach einem der vorhergehenden Ansprüche, **dadurch gekennzeichnet, dass** das Markierungselement (22) aus einer Farbpaste besteht, die eine röntgendichte Substanz wie z. B. Bariumsulfat enthält.

10. Mittel nach Anspruch 9, **dadurch gekennzeichnet, dass** die Farbpaste mittels Siebdruck, Tampondruck oder Sprühtechnik auf die Folie (18) aufgebracht ist.

11. Mittel nach einem der Ansprüche 1 bis 8, **dadurch gekennzeichnet, dass** das Markierungselement (22) aus Metallfäden oder Metallkettchen besteht.

## Claims

1. Means for marking parts of the body to be medically checked or radiologically treated, comprising a marker element (22) to be applied onto said part of the body, said marker element containing a substance opaque to X-rays and attached to a transparent and flexible foil (18) one surface of which is coated with an adhesive layer (26) to be sticked onto said part of the body (20), **characterized in that** said foil (18) is covered, on its surface coated with said adhesive layer (26), by a strippable protective silicone film (28) whereas its opposite surface is firmly fixed on a flat backing (12) having an opening (14) in the area of said foil (18).

2. Means according to claim 1 wherein said foil (18) is provided within the area of the opening (14) with a circumferential perforating rule (30).

3. Means according to claim 1 or 2, wherein said opening (14) has a circular form.

4. Means according to any of the preceding claims, wherein said backing (12) consists of a section made of paper, cardboard or plastics.

5. Means according to any of the preceding claims, wherein said marker element (22) is enclosed between the transparent adhesive layer (26) and said foil (18).

6. Means according to any of the preceding claims, wherein said backing (12) has a rectangular, preferably square form.

7. Means according to any of the preceding claims, wherein said marker element (22) is cross-shaped having two orthogonal legs (24) intersecting in the centre of said opening (14).

8. Means according to claim 7, wherein the backing (12), on the perimeter of said opening (14), is provided with four reference lines (34) lying on the bisecting lines of said cross.

9. Means according to any of the preceding claims, wherein said marker element (22) consists of a colour paste containing a substance opaque to X-rays, e. g. barium sulphate.

10. Means according to claim 9, wherein said colour paste is applied to said foil (18) by means of serigraphy, tamper printing or spray coating.

11. Means according to any of the preceding claims 1 to 8, wherein said marker element (22) consists of metal filaments or small metallic chains.

## Revendications

1. Moyen pour marquer des parties du corps, pour le traitement ou le diagnostique radiologique, par un élément marquant (22) à appliquer sur la surface du corps, cet élément contenant une substance opaque aux rayons X et supporté par une feuille souple et transparente (18) munie sur une des faces d'une couche adhésive (26) pour être appliquée sur la partie du corps (20), **caractérisé par le fait que** la feuille (18) est recouverte sur sa face collante d'une feuille de protection (28) détachable en silicone tandis que sa face opposée est reliée à un élément de support plat (12) dans lequel est pratiqué une fenêtre (14) positionnée dans la zone de la feuille (18).

2. Moyen selon la revendication 1 **caractérisé par le fait que** la feuille (18) présente sur son bord une perforation (13) située à l'intérieur de la fenêtre (14).

3. Moyen selon la revendication 1 ou 2 **caractérisé par le fait que** la fenêtre (15) est de forme circulaire.

4. Moyen selon une des revendications précédentes 1 **caractérisé par le fait que** l'élément de support (20) est composé d'une découpe en matière synthétique, papier ou carton.

5. Moyen selon une des revendications précédentes **caractérisé par le fait que** l'élément marquant (22) est inséré entre la couche collante transparente (26) et la feuille (18).

6. Moyen selon une des revendications précédentes **caractérisé par le fait que** l'élément de support (12) est rectangulaire ou de préférence carré.

7. Moyen selon une des revendications précédentes 1 **caractérisé par le fait que** l'élément marquant (22) est en forme de croix à branches (27) perpendiculaires qui se croisent au centre de la fenêtre (14).

8. Moyen selon la revendication 7 **caractérisé par le fait que** le bord extérieur de la fenêtre (14) de l'élément de support (12) est marqué de quatre repères de référence (34) positionnés sur les bissectrices des angles de la croix.

9. Moyen selon une des revendications précédentes **caractérisé par le fait que** l'élément marquant (22) est composé d'une encre de couleur comprenant une substance opaque aux rayons X, par exemple le sulfate de baryum.

10. Moyen selon la revendication 9 **caractérisé par le fait que** l'encre de couleur est appliquée sur la feuille (18) par sérigraphie ou par tampon ou par pulvérisation.

11. Moyen selon une des revendications 1 à 8 **caractérisé par le fait que** l'élément marquant (22) est composé de fils métalliques ou de chaînettes métalliques.
